(19) Europäisches Patentamt
European Patent Office
Office européen des brevets

(11) Veröffentlichungsnummer: **0 046 450**
**B1**

# (12) EUROPÄISCHE PATENTSCHRIFT

(45) Veröffentlichungstag der Patentschrift: **15.02.84**

(51) Int. Cl.³: **C 07 C 153/05,** A 01 N 47/30

(21) Anmeldenummer: **81810328.5**

(22) Anmeldetag: **13.08.81**

## (54) Thiocarbamoylalkoxy-phenyl-harnstoffe mit Herbizidwirkung, ihre Herstellung und Verwendung.

(30) Priorität: **19.08.80 CH 6254/80**

(43) Veröffentlichungstag der Anmeldung: **24.02.82 Patentblatt 82/8**

(45) Bekanntmachung des Hinweises auf die Patenterteilung: **15.02.84 Patentblatt 84/7**

(84) Benannte Vertragsstaaten: **BE CH DE FR GB LI NL**

(56) Entgegenhaltungen: **DE - A - 2 044 735**

(73) Patentinhaber: **CIBA-GEIGY AG, Patentabteilung Postfach, CH-4002 Basel (CH)**

(72) Erfinder: **Berrer, Dagmar, Dr., Steingrubenweg 92, CH-4125 Riehen (CH)**
Erfinder: **Pissiotas, Georg, Dr., Breslauerstrasse 8, D-7850 Lörrach (DE)**
Erfinder: **Rohr, Otto, Dr., Kilbertweg 19, CH-4106 Therwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Thiocarbamoylalkoxy-phenyl-harnstoffe mit Herbizidwirkung, ihre Herstellung und Verwendung

Die vorliegende Erfindung betrifft neue Thiocarbamoylalkoxy-phenyl-harnstoffe mit selektiver herbizider Wirkung, deren Herstellung, sie enthaltende Mittel sowie deren Verwendung zur Bekämpfung von Unkräutern in Nutzpflanzenkulturen wie Soja, Baumwolle, Getreide, Mais, Hirse und Reis.

Die Thiocarbamoylalkoxy-phenyl-harnstoffe entsprechen der allgemeinen Formel

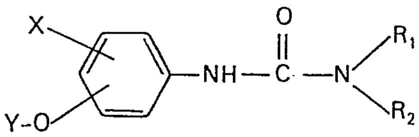

(I)

worin

X Wasserstoff, Halogen, $C_1$–$C_2$-Halogenalkoxy oder $C_1$–$C_2$-Halogenalkyl,

Y eine Thiocarbamoylalkylgruppe der Formel

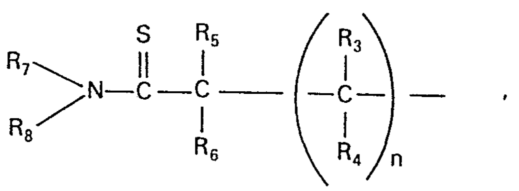

,

n Null, eins oder zwei,

$R_1$ Wasserstoff, $C_1$–$C_4$-Alkyl, $C_2$–$C_4$-Alkenyl, $C_2$–$C_4$-Alkinyl oder $C_1$–$C_4$-Alkoxy,

$R_2$ Wasserstoff, $C_1$–$C_4$-Alkyl oder $C_2$–$C_4$-Alkenyl,

$R_3$, $R_4$ und $R_6$ unabhängig voneinander jeweils Wasserstoff oder $C_1$–$C_4$-Alkyl,

$R_5$ Wasserstoff, $C_1$–$C_4$-Alkyl, $C_1$–$C_4$-Alkoxy oder $C_2$–$C_5$-Alkoxyalkyl,

$R_7$ Wasserstoff, $C_1$–$C_4$-Alkyl, $C_2$–$C_4$-Alkenyl, $C_2$–$C_4$-Alkinyl, $C_1$–$C_4$-Alkoxy oder $C_1$–$C_2$-Alkylphenyl und

$R_8$ Wasserstoff oder $C_1$–$C_4$-Alkyl bedeuten, wobei die Reste $R_1$, und $R_2$ oder $R_7$ und $R_8$ zusammen mit dem sie tragenden Stickstoffatom einen gegebenenfalls durch weitere Heteroatome unterbrochenen 5- bis 6gliedrigen Heterocyclus bilden können.

In der Definition von X sind unter Halogen, Fluor, Chlor oder Brom, insbesondere aber Fluor und vor allem Chlor zu verstehen.

Beispiele für Alkyl sind Methyl, Äthyl, n-Propyl, i-Propyl sowie die vier isomeren Butylreste, vorzugsweise jedoch Methyl oder Äthyl.

Alkoxyreste sind zum Beispiel Methoxy, Äthoxy, n-Propyloxy i-Propyloxy oder tert. Butyloxy.

Sinngemäss sind unter Halogenalkyl und Halogenalkoxy im allgemeinen zu verstehen: Fluormethyl, Fluormethoxy, Chlormethyl, α-Chloräthyl, β-Chloräthyl, Trifluormethyl, Trifluormethoxy, α,α,β,β-Tetrachloräthyl oder β,β,γ,γ,-Tetrafluorpropyl, vor allem aber Chlormethyl, Trifluormethyl oder Trifluormethoxy.

Beispiele für Alkenyl sind Vinyl, Allyl, Methallyl oder Crotyl, insbesondere aber Vinyl oder Allyl.

Unter Alkinyl sind im gleichen Sinn Äthinyl und insbesondere Propargyl zu verstehen.

Beispiele für mögliche Stickstoffheterocyclen sind Pyrrolidin, Pyrrolin, Imidazolidin, Pyrazolidin, Piperidin, Piperazin, Morpholin, Pyrazolin, Imidazol, Pyrrol oder Imidazolin, vorzugsweise aber Pyrrolidin, Piperidin oder Morpholin.

Wegen ihrer Wirkung bevorzugt sind die Verbindungen der Formel I, in denen der Substituent Y-O- die meta- oder para-Stellung des Phenylkerns besetzt.

Hiervon sind von besonderer Bedeutung diejenigen Verbindungen, in denen der Rest Y-O- die meta-Stellung des Phenylkerns besetzt.

Weiterhin bevorzugt sind die Verbindungen der Formel I, in denen n Null ist.

Ebenso im Vordergrund stehen die Verbindungen der Formel I in denen X Wasserstoff oder Halogen bedeutet.

Von überragendem Interesse sind jedoch die Verbindungen der Formel I, in denen der Substituent Y-O- die meta-Stellung des Phenylkerns besetzt; n Null ist; die Reste X, $R_7$ und $R_8$ Wasserstoff; $R_2$ Methyl und $R_1$ Methoxy oder Methyl bedeuten.

Die Herstellung der Verbindungen der Formel I erfolgt nach an sich bekannten Methoden.

So werden die Thiocarbamoyl-Verbindungen der Formel I analog zu einem in Houben-Weyl, Methoden der organischen Chemie, 4. Aufl., Band 8, S. 672 beschriebenen Verfahren erhalten, indem man ein Carbonsäurenitril der Formel II

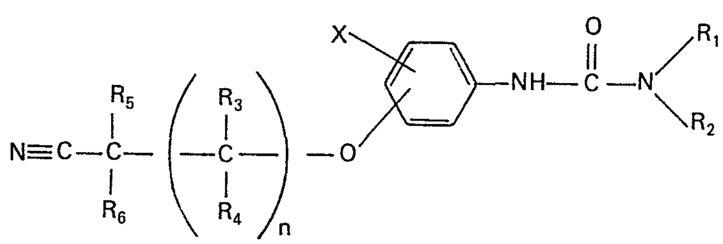

(II)

worin X, n und $R_1$ bis $R_6$ die unter Formel I gegebene Bedeutung haben, gegebenenfalls in Gegenwart eines Katalysators mit Schwefelwasserstoff umsetzt.

Gewünschtenfalls kann man die Thiocarbonsäureamide nach allgemein bekannten Methoden in die N-substituierten Amide überführen.

Die Umsetzungen erfolgen in inerten organischen Lösungsmitteln wie z.B. Methanol, Äthanol, Tetrahydrofuran, Dioxan, Diäthyläther, Benzol, Pyridin und Toluol bei Raumtemperatur unter Normaldruck. Mit Vorteil kann man die Reaktionen aber auch in verflüssigtem Schwefelwasserstoff unter erhöhter Temperatur (30–150°) im Autoklaven ausführen.

Als geeignete Katalysatoren haben sich basische Katalysatoren wie z.B. Ammonium- oder Alkalibisulfide, aber auch organische Basen wie Diäthylamin, Triäthylamin, Piperidin, Pyrrolidin, Pyridin, Morpholin, Chinolidin und 4-Dimethylaminopyridin erwiesen.

Die Ausgangsverbindungen der Formel II sind nach den in der europäischen Patentanmeldung 8 0810 090.3 beschriebenen Verfahren erhältlich.

So erhält man z.B. die Ausgangsverbindungen der Formel II, indem man eine entsprechend substituierte Phenylverbindung der Formel III

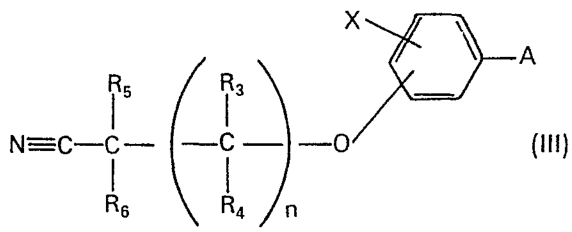

(III)

mit einem Amin der Formel IV

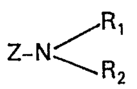

(IV)

umsetzt. In den Formeln III und IV haben X, n und $R_1$ bis $R_6$ die unter Formel I angegebene Bedeutung, während A und Z Reste darstellen, die unter Anlagerung oder Kondensation Harnstoffe bilden. Dabei stellt eine der Gruppen A und Z ein Amin dar, die andere eine Alkoxycarbonyl-, Haloformyl-, Carbamoylgruppe oder insbesondere den Isocyanatrest.

Ferner erhält man die Ausgangsmaterialien durch Umsetzen von Hydroxyphenyl-harnstoffen der Formel V

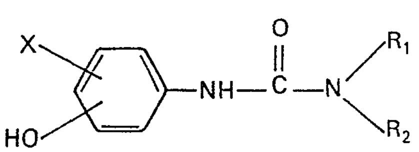

(V)

mit Cyanoalkylhalogeniden der Formel VI

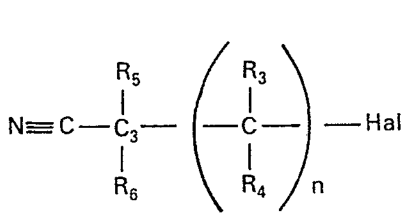

(VI)

in Gegenwart eines säurebindenden Mittels.

In den Formeln V und VI haben X, n und $R_1$ bis $R_6$ die unter Formel I angegebene Bedeutung und Hal bedeutet Chlor oder Brom.

Die neuen Wirkstoffe der Formel I sind stabile Verbindungen, welche in üblichen organischen Lösungsmitteln, wie Alkoholen, Äthern, Ketonen, Dimethylformamid und Dimethylsulfoxyd löslich sind.

Die Verbindungen der Formel I weisen ausgesprochen selektiv-herbizide Eigenschaften im allgemeinen auf und erweisen sich als besonders vorteilhaft zum Bekämpfen von Unkräutern sowohl bei pre-emergenter als auch post-emergenter Anwendung in Kulturen von Nutzpflanzen, insbesondere in Kulturen von Soja, Baumwolle, Getreide, Mais, Hirse und Reis.

Die Wirkstoffe weisen dabei in der post-emergenten Anwendung selektive herbizide Eigenschaften gegen einige Monokotyledonen auf. Hervorragende Ergebnisse werden jedoch bei der Nachauflaufanwendung gegen Dikotyledonen erzielt. Das Hauptanwendungsgebiet liegt deshalb bei der postemergenten Bekämpfung von dikotylen Unkräutern, besonders einzelner Problemunkräuter wie Galium aparine.

Besonders bemerkenswert ist, dass Getreide wie Weizen und Gerste unwesentlich und Mais und Hirse kaum in ihrem Wachstum beeinträchtigt werden. Bei vorsichtiger Dosierung lässt sich mit den Verbindungen der Formel I auch in Soja ein gutes selektives Wirkungsbild gegen dikotyle Unkräuter erzielen.

Die Handhabung der Verbindungen der Formel I bedarf keiner vorsorglicher Massnahmen. Ihre Formulierung als flüssige herbizide Mittel gelingt nur mit Hilfe üblicher Lösungsvermittler und/oder Dispergiermittel.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Homogengranulate;

In Wasser dispergierbare Wirkstoffkonzentrate:
Spritzpulver (wettable powder), Pasten, Emulsionen; Emulsionskonzentrate;

Flüssige Aufarbeitungsformen: Lösungen.

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen. Dabei können die Aufwandmengen in weiten Grenzen schwanken, z.B. zwischen 0,1 bis 10 kg Wirkstoff pro Hektar, vorzugsweise werden jedoch 0,5 bis 5 kg Wirkstoff pro Hektar eingesetzt.

Zur Verbreiterung des Wirkungsspektrums oder zur Erreichung eines gewünschten synergistischen oder auch antagonistischen Effektes können diese Verbindungen auch gemeinsam mit bekannten herbiziden, insektiziden, nematoziden, bakteriziden, nematoziden, akariziden oder fungiziden Verbindungen eingesetzt werden. Als Kombinationspartner kommen beispielsweise die im «Pesticide Manual» 6th, Edition,

The British Crop Protection Council, ISBN 0-901 436-44-5, beschriebenen Präparate in Frage.

In den folgenden Beispielen sind die Temperaturen in Celsiusgraden angegeben. Teile und Prozentangaben beziehen sich auf das Gewicht.

Beispiel 1

α-[m-(3-Methoxy-3-methyl-ureido)-phenoxy]-thiopropionsäureamid.

Durch ein Gemisch von 17,5 g α-(m-(3-Methoxy-3-methyl-ureido-phenoxy)-propionitril, 24 g Pyridin und 7 g Triäthylamin wird unter Rühren und Kühlung auf 20-30°C für eine Stunde ein Strom von Schwefelwasserstoff geleitet. Das Reaktionsgemisch wird in Wasser gegossen. Durch Extraktion mit Äthylacetat, Trocknen und Eindampfen der organischen Phase und Kristallisation des Rückstandes aus Äthanol/Wasser erhält man 12,3 g α-[m-(3-Methoxy-3-methylureido)-phenoxy]-thiopropionsäureamid vom Schmelzpunkt 95-96°C (Verbindung 2).

In der folgenden Tabelle sind die nach dem Beispiel hergestellte Verbindung 2 und analog synthetisierte Thiocarbamoylalkoxy-phenylharnstoffe der Formel I aufgelistet.

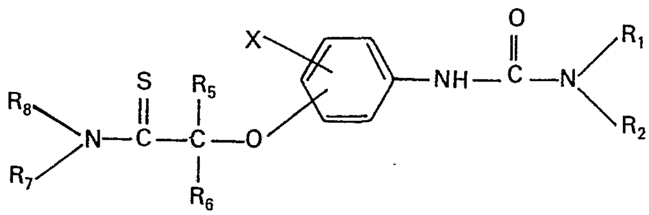

| Nr. | X | $R_1$, | $R_2$ | $R_5$ | $R_6$ | $R_7$, | $R_8$ | Stellung des Thiocarbamoyl-alkoxyrestes | phys. Daten |
|---|---|---|---|---|---|---|---|---|---|
| 1 | H | $CH_3$, | $CH_3$ | H | H | H, | H | 3 | Smp. 166° |
| 2 | H | $OCH_3$, | $CH_3$ | $CH_3$ | H | H, | H | 3 | Smp. 95-96° |
| 3 | H | $CH_3$, | $CH_3$ | $CH_3$ | H | H, | H | 3 | $n_d^{50}$: 1,5839 |
| 4 | H | $CH_3$, | $CH_3$ | $C_2H_5$ | H | H, | H | 3 | Smp. 62° |
| 5 | H | $OCH_3$, | $CH_3$ | $C_2H_5$ | H | H, | H | 3 | Smp. 123° |
| 6 | H | $C_4H_9n$, | H | $CH_3$ | H | $C_4H_9n$, | H | 3 | $n_d^{30}$: 1,5539 |
| 7 | 3-Cl | $CH_3$, | $CH_3$ | $CH_3$ | H | H, | H | 4 | Smp. 180–181° |
| 8 | 3-Cl | $CH_3$, | $CH_3$ | H | H | H, | H | 4 | Smp. 175–176° |
| 9 | 3-Cl | $OCH_3$, | $CH_3$ | H | H | H, | H | 4 | Smp. 173–174° |
| 10 | 3-Cl | $CH_3$, | $CH_3$ | $C_2H_5$ | H | H, | H | 4 | Smp. 175° |
| 11 | 3-Cl | $OCH_3$, | $CH_3$ | $CH_3$ | H | H, | H | 4 | Smp. 186–187° |
| 12 | H | $OCH_3$, | $CH_3$ | $CH_3$ | H | H, | H | 4 | Smp. 185° |
| 13 | H | $CH_3$, | $CH_3$ | $CH_3$ | H | H, | H | 4 | Smp. 174–175° |
| 14 | H | $CH_3$, | $CH_3$ | H | H | H, | H | 4 | Smp. 181–182° |
| 15 | 3-Cl | $OCH_3$, | $CH_3$ | $C_2H_5$ | H | H, | H | 4 | Smp. 184–185° |
| 16 | H | $OCH_3$, | $CH_3$ | $C_2H_5$ | H | H, | H | 4 | Smp. 136–137° |
| 17 | H | $OCH_3$, | $CH_3$ | $CH_3$ | H | $CH_3$, | H | 3 | Smp. 97–98° |
| 18 | H | $OCH_3$, | $CH_3$ | $CH_3$ | H | $C_2H_5$, | H | 3 | Öl |
| 19 | H | $OCH_3$, | $CH_3$ | $CH_3$ | H | $C_3H_7n$, | H | 3 | Smp. 133–134° |
| 20 | H | $OCH_3$, | $CH_3$ | $CH_3$ | H | $C_3H_7i$, | H | 3 | |
| 21 | H | $OCH_3$, | $CH_3$ | $CH_3$ | H | $C_4H_9n$, | H | 3 | $n_D^{39}$: 1,5586 |
| 22 | H | $OCH_3$, | $CH_3$ | $CH_3$ | H | $C_4H_9i$, | H | 3 | |
| 23 | H | $OCH_3$, | $CH_3$ | $CH_3$ | H | $C_4H_9s$, | H | 3 | |
| 24 | H | $OCH_3$, | $CH_3$ | $CH_3$ | H | $C_4H_9t$, | H | 3 | |
| 25 | H | $OCH_3$, | $CH_3$ | $CH_3$ | H | $-CH_2-CH=CH_2$, | H | 3 | |
| 26 | H | $OCH_3$, | $CH_3$ | $CH_3$ | H | $CH_3$, | $CH_3$ | 3 | |
| 27 | H | $OCH_3$, | $CH_3$ | $CH_3$ | H | $OCH_3$, | $CH_3$ | 3 | |
| 28 | H | $OCH_3$, | $CH_3$ | $CH_3$ | H | $C_4H_9n$, | $CH_3$ | 3 | |
| 29 | H | $OCH_3$, | $CH_3$ | $CH_3$ | H | $C_4H_9n$, | $C_4H_9n$ | 3 | |
| 30 | H | $OCH_3$, | $CH_3$ | $CH_3$ | H | $C_2H_5$, | $C_2H_5$ | 3 | |
| 31 | H | $OCH_3$, | $CH_3$ | $CH_3$ | H | $-(CH_2)_2-O-(CH_2)_2-$ | | 3 | |
| 32 | H | $OCH_3$, | $CH_3$ | $CH_3$ | H | 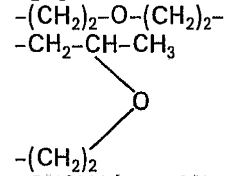 | | 3 | |
| 33 | H | $OCH_3$, | $CH_3$ | $CH_3$ | H | $-CH(CH_3)-C\equiv CH$, | $CH_3$ | 3 | |
| 34 | H | $CH_3$, | $CH_3$ | $CH_3$ | H | $CH_3$, | H | 3 | Smp. 177–118° |
| 35 | H | $CH_3$, | $CH_3$ | $CH_3$ | H | $C_2H_5$, | H | 3 | Smp. 107–108° |
| 36 | H | $CH_3$, | $CH_3$ | $CH_3$ | H | $C_3H_7n$, | H | 3 | Smp. 91–92° |

| Nr. | X | $R_1$, | $R_2$ | $R_5$ | $R_6$ | $R_7$, | $R_8$ | Stellung des Thiocarbamoyl-alkoxyrestes | phys. Daten |
|---|---|---|---|---|---|---|---|---|---|
| 37 | H | $CH_3$, | $CH_3$ | $CH_3$ | H | $C_3H_7i$, | H | 3 | Smp. 133–134° |
| 38 | H | $CH_3$, | $CH_3$ | $CH_3$ | H | $C_4H_9n$, | H | 3 | |
| 39 | H | $CH_3$, | $CH_3$ | $CH_3$ | H | $C_4H_9i$, | H | 3 | |
| 40 | H | $CH_3$, | $CH_3$ | $CH_3$ | H | $C_4H_9s$, | H | 3 | |
| 41 | H | $CH_3$, | $CH_3$ | $CH_3$ | H | $C_4H_9t$, | H | 3 | |
| 42 | H | $CH_3$, | $CH_3$ | $CH_3$ | H | $C_2H_4OCH_3$, | H | 3 | Smp. 101–105° |
| 43 | H | $CH_3$, | $CH_3$ | $CH_3$ | H | $CH_3$ | $CH_3$ | 3 | |
| 44 | H | $CH_3$, | $CH_3$ | $CH_3$ | H | $OCH_3$, | $CH_3$ | 3 | |
| 45 | H | $CH_3$, | $CH_3$ | $CH_3$ | H | $C_4H_9n$, | $CH_3$ | 3 | |
| 46 | H | $CH_3$, | $CH_3$ | $CH_3$ | H | $C_2H_5n$, | $C_2H_5$ | 3 | |
| 47 | H | $CH_3$, | $CH_3$ | $CH_3$ | H | $-CH(CH_3)-C{\equiv}CH$, | $CH_3$ | 3 | |
| 48 | H | $CH_3$, | H | $CH_3$, | H | H, | H | 3 | |
| 49 | H | $CH_3$, | H | $CH_3$ | H | $C_4H_9t$, | H | 3 | |
| 50 | H | $CH_3$, | H | $CH_3$ | H | $C_4H_9i$, | H | 3 | |
| 51 | H | $-CH(CH_3)-C{\equiv}CH$, | $CH_3$ | $CH_3$ | H | H, | H | 3 | |
| 52 | H | $C_4H_9n$, | $CH_3$ | $CH_3$ | H | H, | H | 3 | |
| 53 | H | $CH_3$, | $CH_3$ | $CH_3$ | H | $-(CH_2)_5-$ | | 3 | |
| 54 | H | $CH_3$, | $CH_3$ | $CH_3$ | H | $-(CH_2)_4-$ | | 3 | |
| 55 | H | $CH_3$, | $CH_3$ | $CH_3$ | H | 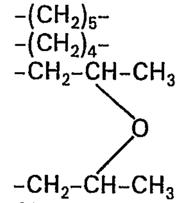 | | 3 | |
| 56 | H | $OCH_3$, | $CH_3$ | $C_2H_5$ | H | $CH_3$, | H | 3 | Smp. 111–112° |
| 57 | H | $OCH_3$, | $CH_3$ | $C_2H_5$ | H | $C_2H_5$, | H | 3 | Harz |
| 58 | H | $OCH_3$, | $CH_3$ | $C_2H_5$ | H | $C_3H_7n$, | H | 3 | |
| 59 | H | $OCH_3$, | $CH_3$ | $C_2H_5$ | H | $C_3H_7i$, | H | 3 | |
| 60 | H | $OCH_3$, | $CH_3$ | $C_2H_5$ | H | $C_4H_9n$, | H | 3 | |
| 61 | H | $OCH_3$, | $CH_3$ | $C_2H_5$ | H | $C_4H_9i$, | H | 3 | |
| 62 | H | $OCH_3$, | $CH_3$ | $C_2H_5$ | H | $C_4H_9s$, | H | 3 | |
| 63 | H | $OCH_3$, | $CH_3$ | $C_2H_5$ | H | $C_4H_9t$, | H | 3 | |
| 64 | H | $OCH_3$, | $CH_3$ | $C_2H_5$ | H | $-CH_2-CH{=}CH_2$, | H | 3 | |
| 65 | H | $OCH_3$, | $CH_3$ | $C_2H_5$ | H | $CH_3$, | $CH_3$ | 3 | |
| 66 | H | $OCH_3$, | $CH_3$ | $C_2H_5$ | H | $OCH_3$, | $CH_3$ | 3 | |
| 67 | H | $OCH_3$, | $CH_3$ | $C_2H_5$ | H | $C_4H_9n$, | $CH_3$ | 3 | |
| 68 | H | $OCH_3$, | $CH_3$ | $C_2H_5$ | H | $C_4H_9n$, | $C_4H_9n$ | 3 | |
| 69 | H | $OCH_3$, | $CH_3$ | $C_2H_5$ | H | $C_2H_5$, | $C_2H_5$ | 3 | |
| 70 | H | $OCH_3$, | $CH_3$ | $C_2H_5$ | H | $-(CH_2)_2-O-(CH_2)_2-$ | | 3 | |

| Nr. | X | $R_1$, | $R_2$ | $R_5$ | $R_6$ | $R_7$, | $R_8$ | Stellung des Thiocarbamoyl-alkoxyrestes | phys. Daten |
|---|---|---|---|---|---|---|---|---|---|
| 71 | H | $OCH_3$, | $CH_3$ | $C_2H_5$ | H | 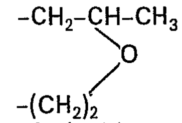 | | 3 | |
| 72 | H | $OCH_3$, | $CH_3$ | $C_2H_5$ | H | $-CH(CH_3)-C\equiv CH$, | $CH_3$ | 3 | |
| 73 | H | $CH_3$, | $CH_3$ | $C_2H_5$ | H | $CH_3$, | H | 3 | |
| 74 | H | $CH_3$, | $CH_3$ | $C_2H_5$ | H | $C_2H_5$, | H | 3 | |
| 75 | H | $CH_3$, | $CH_3$ | $C_2H_5$ | H | $C_3H_7n$, | H | 3 | |
| 76 | H | $CH_3$, | $CH_3$ | $C_2H_5$ | H | $C_3H_7i$, | H | 3 | |
| 77 | H | $CH_3$, | $CH_3$ | $C_2H_5$ | H | $C_4H_9n$, | H | 3 | |
| 78 | H | $CH_3$, | $CH_3$ | $C_2H_5$ | H | $C_4H_9i$, | H | 3 | |
| 79 | H | $CH_3$, | $CH_3$ | $C_2H_5$ | H | $C_4H_9s$, | H | 3 | |
| 80 | H | $CH_3$, | $CH_3$ | $C_2H_5$ | H | $C_4H_9t$, | H | 3 | |
| 81 | H | $CH_3$, | $CH_3$ | $C_2H_5$ | H | $-CH_2-CH=CH_2$, | H | 3 | |
| 82 | H | $CH_3$, | $CH_3$ | $C_2H_5$ | H | $CH_3$, | $CH_3$ | 3 | |
| 83 | H | $CH_3$, | $CH_3$ | $C_2H_5$ | H | $OCH_3$, | $CH_3$ | 3 | |
| 84 | H | $CH_3$, | $CH_3$ | $C_2H_5$ | H | $C_4H_9n$, | $CH_3$ | 3 | |
| 85 | H | $CH_3$, | $CH_3$ | $C_2H_5$ | H | $C_2H_5$, | $C_2H_5$ | 3 | |
| 86 | H | $CH_3$, | $CH_3$ | $C_2H_5$ | H | $-CH(CH_3)-C\equiv CH$, | $CH_3$ | 3 | |
| 87 | H | $CH_3$, | H | $C_2H_5$ | H | H, | H | 3 | |
| 88 | H | $CH_3$, | H | $C_2H_5$ | H | $C_4H_9t$, | H | 3 | |
| 89 | H | $CH_3$, | H | $C_2H_5$ | H | $C_4H_9i$, | H | 3 | |
| 90 | H | $-CH(CH_3)-C\equiv CH$, | $CH_3$ | $C_2H_5$ | H | H, | H | 3 | |
| 91 | H | $C_4H_9n$, | $CH_3$ | $C_2H_5$ | H | H, | H | 3 | |
| 92 | H | $CH_3$, | $CH_3$ | $C_2H_5$ | H | $-(CH_2)_5-$ | | 3 | |
| 93 | H | $CH_3$, | $CH_3$ | $C_2H_5$ | H | $-(CH_2)_4-$ | | 3 | |
| 94 | H | $CH_3$, | $CH_3$ | $C_2H_5$ | H | 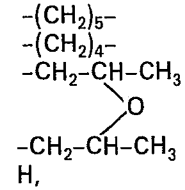 | | 3 | |
| 95 | H | $CH_3$, | $CH_3$ | $C_3H_7n$ | H | H, | H | 3 | |
| 96 | H | $OCH_3$, | $CH_3$ | $C_3H_7n$ | H | H, | H | 3 | Smp. 86–87° |
| 97 | H | $CH_3$, | $CH_3$ | $C_3H_7n$ | H | $C_4H_9n$, | H | 3 | |
| 98 | H | $CH_3$, | $CH_3$ | $C_3H_7n$ | H | $CH_3$, | H | 3 | Smp. 132–134° |
| 99 | H | $OCH_3$, | $CH_3$ | $C_3H_7n$ | H | $C_4H_9n$, | H | 3 | |
| 100 | H | $OCH_3$, | $CH_3$ | $C_3H_7n$ | H | $CH_3$, | H | 3 | Smp. 107–110° |
| 101 | 3-$CF_3$ | $CH_3$, | $CH_3$ | $CH_3$ | H | H, | H | 4 | Smp. 179–180° |
| 102 | 3-$CF_3$ | $OCH_3$, | $CH_3$ | $CH_3$ | H | H, | H | 4 | Smp. 178–180° |
| 103 | H | $CH_3$, | $CH_3$ | $C_2H_5$ | H | H, | H | 4 | Smp. 168–169° |
| 104 | H | $CH_3$, | $CH_3$ | $CH_3$ | $CH_3$ | H, | H | 3 | Smp. 89–90° |

| Nr. | X | $R_{1'}$ | $R_2$ | $R_5$ | $R_6$ | $R_{7'}$ | $R_8$ | Stellung des Thiocarbamoyl-alkoxyrestes | phys. Daten |
|---|---|---|---|---|---|---|---|---|---|
| 105 | H | $OCH_3$, | $CH_3$ | $CH_3$ | $CH_3$ | H, | H | 3 | Smp. 143–144° |
| 106 | H | $OCH_3$, | $CH_3$ | $C_2H_5$ | H | H, | H | 4 | |
| 107 | H | $OCH_3$, | $CH_3$ | H | H | H, | H | 4 | Smp. 154–155° |
| 108 | H | $OCH_3$, | $CH_3$ | $C_3H_7n$ | H | H | H | 3 | Smp. 86–87° |
| 110 | H | $OCH_3$, | $CH_3$ | $C_3H_7i$ | H | H | H | 3 | $n_D^{40}$: 1.5822 |
| 111 | H | $OCH_3$, | $CH_3$ | H | H | H | H | 3 | Smp. 180° |
| 112 | H | $OCH_3$, | $CH_3$ | $CH_3$ | $CH_3$ | $CH_3$ | H | 3 | Smp. 94–95° |
| 113 | 4–Cl | $OCH_3$, | $CH_3$ | $CH_3$ | H | $C_2H_5$ | H | 3 | Smp. 114–116° |
| 114 | 4–Cl | $CH_3$, | $CH_3$ | $CH_3$ | H | $CH_3$ | H | 3 | Smp. 94–95° |
| 115 | 3–Cl | $OCH_3$, | $CH_3$ | H | H | △- | H | 4 | Smp. 136–138° |
| 116 | 3–Cl | $OCH_3$, | $CH_3$ | $CH_3$ | H | $CH_3$ | H | 4 | Smp. 119-120° |
| 117 | 3–Cl | $CH_3$, | $CH_3$ | $CH_3$ | H | $C_2H_4OCH_3$ | H | 4 | Smp. 67–71° |
| 118 | 3–$CF_3$ | $CH_3$, | $CH_3$ | $C_2H_5$ | H | H | H | 4 | Smp. 160–162° |
| 119 | 3–$CF_3$ | $OCH_3$, | $CH_3$ | $C_2H_5$ | H | H | H | 4 | Smp.160–162° |
| 120 | 3–$CF_3$ | $CH_3$, | $CH_3$ | $CH_3$ | H | $CH_3$ | H | 4 | Smp. 157–161° |

Beispiel 2

Spritzpulver

Zur Herstellung eines a) 70%igen und b) 10%igen Spritzpulvers werden folgende Bestandteile verwendet:

a) 70 Teile m-(3,3-Dimethyl-ureido)-phenoxy-thioacetamid,
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Kreide;

b) 10 Teile des obigen Wirkstoffs,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1–80% Wirkstoff erhalten werden, die sich zur Unkrautbekämpfung in Pflanzenkulturen eignen.

Beispiel 3

Paste

Zur Herstellung einer 45%igen Paste werden folgende Stoffe verwendet:

45 Teile α-[m-(3-Methyl-3-methoxy-ureido)-phenoxy]-thiopropionsäureamid,
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Beispiel 4

Emulsionskonzentrat

Zur Herstellung eines 25%igen Emulsionskonzentrates werden

25 Teile α-[m-(3-Methoxy-3-methyl-ureido)-phenoxy]-thio-propionsäureamid,
10 Teile einer Mischung von Nonylphenolpolyoxyäthylen oder Calciumdidecylbenzolsulfonat,

10 Teile Cyclohexanon,
55 Teile Xylol

miteinander vermischt. Dieses Konzentrat kann mit Wasser zu Emulsionen auf geeignete Konzentrationen von z.B. 0,1% verdünnt werden. Solche Emulsionen eignen sich zur Bekämpfung von Unkräutern in Kulturpflanzungen.

Beispiel 5
Flowable

Zur Herstellung eines 45%igen Flowables werden folgende Stoffe verwendet:

45 Teile α-[m-(3,3-Dimethyl-ureido)-phenoxy]-n-thiobutter-säureamid,
5 Teile Octylphenoxypolyäthylenglycol mit 9-10 Mol Äthylenoxyd pro Mol Octylphenol,
3 Teile von einem Gemisch aromatischer Sulfonsäuren, kondensiert mit Formaldehyd als Ammoniumsalz,
1 Teil Siliconöl in Form einer 75%igen Emulsion,
0,1 Teil einer Mischung von 1-(3-Chlorallyl)-3,5,7-tri-azo-azonium-adamantan-chlorid mit Natriumcarbonat, Chloridwert mind. 11,5%,
0,2 Teile eines biopolymeren Verdickers mit max. 100 Keimen pro Gramm,
42,7 Teile Wasser,
5 Teile Äthylenglykol.

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Beispiel 6

Nachweis der Herbizidwirkung vor dem Auflaufen der Pflanzen.

Im Gewächshaus werden Pflanzensamen in Blumentöpfe von 12-15 cm Durchmesser gesät. Unmittelbar danach wird die Erdoberfläche mit einer wässrigen Dispersion oder Lösung der Wirkstoffe, erhalten aus einem Flowable oder Spritzpulver, behandelt. Es werden verschiedene Konzentrationen mit Wirkstoffmengen pro Hektar angewendet. Die Töpfe werden im Gewächshaus bei einer Temperatur von 22-25°C und 50-70% relativer Luftfeuchtigkeit gehalten. Nach 3 Wochen wird der Versuch ausgewertet.

Die Wirkung wird nach folgender Skala beurteilt:

1: Pflanzen abgestorben
2 bis 8: Zwischenstufen der Schädigung
9: keine Wirkung – wie unbehandelte Kontrollpflanzen.
–: Pflanze nicht geprüft.

Die Resultate sind in der folgenden Tabelle zusammengefasst.

| Verbindung No. | 2 | 3 | 4 | 5 | 104 | 105 | 108 |
|---|---|---|---|---|---|---|---|
| Aufwandmenge in kg pro Hektar | 4 2 1 ½ | 4 2 1 ½ | 4 2 1 ½ | 4 2 1 ½ | 4 2 1 ½ | 4 2 1 ½ | 4 2 1 ½ |
| Pflanze | | | | | | | |
| Weizen | 1 2 8 9 | 3 4 8 9 | 3 3 6 6 | 2 2 6 6 | 2 2 3 5 | 1 1 2 6 | 2 3 7 8 |
| Mais | 2 6 7 8 | 3 6 9 9 | 3 3 4 4 | 4 7 9 9 | 2 3 7 9 | 2 5 9 9 | 4 9 9 9 |
| Kulturhirse | 6 7 9 9 | 9 9 9 9 | 4 7 9 9 | 4 4 9 9 | 1 3 4 9 | 1 2 6 9 | 6 7 9 9 |
| Soja | 2 2 4 7 | 4 9 9 9 | 3 3 8 9 | 6 7 9 9 | 1 1 3 7 | 1 3 7 9 | 6 7 9 9 |
| Baumwolle | 1 8 – – | 9 9 9 9 | 7 8 9 9 | 5 9 9 9 | 5 9 9 9 | 1 4 9 9 | 9 9 9 9 |
| avena fatua | 1 2 6 9 | 2 2 7 8 | 1 1 1 1 | 1 1 1 2 | 1 2 3 7 | 1 1 2 3 | 1 1 3 7 |
| bromus tectorum | 1 1 5 7 | 1 2 4 4 | 1 1 1 2 | 1 2 2 3 | 1 2 3 4 | 1 1 2 7 | 2 2 4 4 |
| lolium perenne | 1 2 3 8 | 2 3 6 7 | 2 2 3 4 | 1 2 2 6 | 1 2 3 5 | 1 1 1 3 | 1 1 3 8 |
| alopecurus myos. | 1 2 2 3 | 2 2 3 9 | 2 2 2 2 | 1 1 2 2 | 2 2 3 9 | 1 1 3 4 | 2 2 9 9 |
| digitaria sang. | 1 1 2 2 | 1 1 1 2 | 1 2 2 2 | 1 3 8 9 | 1 1 1 2 | 2 3 3 4 | 2 2 7 9 |
| echinochloa crus galli | 2 2 3 4 | 3 8 9 9 | 1 1 1 1 | 1 2 4 4 | 1 1 2 3 | 1 1 2 6 | 1 2 6 9 |
| abutilon | 1 1 1 1 | 1 1 1 1 | 1 1 1 1 | 1 1 1 1 | 1 1 1 1 | 1 1 1 2 | 1 1 3 3 |
| sida spinosa | 1 1 1 1 | 1 1 2 3 | 1 1 1 1 | 1 1 1 1 | 1 1 1 1 | 1 1 1 1 | 1 1 2 2 |
| amarantus retroflexus | 1 1 1 1 | 1 1 1 1 | 1 1 1 1 | 1 1 1 1 | 1 1 1 1 | 1 1 1 1 | 1 1 1 1 |
| chenopodium album | – – – – | – – – – | – – – – | – – – – | 2 2 2 2 | 1 1 4 4 | 1 1 1 1 |
| solanum nigrum | 1 1 1 1 | 1 1 1 1 | 1 1 1 1 | 1 1 1 1 | 1 1 1 1 | 1 1 1 1 | 1 1 4 4 |
| ipomoea purpurea | 1 1 1 3 | 1 1 9 9 | 1 1 1 4 | 1 2 7 9 | 1 1 1 1 | 1 1 1 2 | 1 2 5 9 |
| sinapis alba | 1 1 1 1 | 1 1 4 7 | 1 1 1 1 | 1 1 1 1 | 1 1 2 4 | 1 1 1 1 | 1 1 1 1 |
| stellaria media | 1 1 1 1 | 1 1 1 1 | 1 1 1 1 | 1 1 1 1 | 1 1 1 1 | 1 1 1 1 | 1 1 1 1 |
| chrysanthemum leuc. | 1 1 1 1 | 1 1 1 1 | 1 1 1 1 | 1 1 1 1 | 1 1 1 1 | 1 1 1 1 | 1 1 3 3 |
| portulacca | 1 1 1 1 | 1 1 1 1 | 1 1 1 1 | 1 1 1 1 | – – – – | – – – – | – – – – |
| kochia scop. | 1 1 1 1 | 1 1 1 1 | 1 1 1 1 | 1 1 1 1 | – – – – | – – – – | – – – – |
| sesbania exaltata | 1 1 1 1 | 1 1 2 9 | 1 1 1 1 | 2 7 9 9 | – – – – | – – – – | – – – – |

Beispiel 7

Nachweis der Herbizidwirkung nach Auflaufen der Pflanzen.

Eine grössere Anzahl Unkräuter und Kulturpflanzen, sowohl monocotyle wie dicotyle, wurden nach dem Auflaufen, im 4- bis 6-Blattstadium mit einer wässrigen Wirkstoffdispersion in verschiedenen Dosierungen auf die Pflanzen gespritzt und diese bei 24° bis 26°C und 45–60% relativer Luftfeuchtigkeit gehalten. 15 Tage nach der Behandlung wird der Versuch ausgewertet. Die Resultate sind in der folgenden Tabelle zusammengefasst.

| Verbindung No. | 2 | 3 | 4 | 5 | 10 | 15 | 18 | 104 | 105 | 108 | 110 |
|---|---|---|---|---|---|---|---|---|---|---|---|
| Aufwandmenge in kg pro Hektare | 2 1 ½ | 2 1 ½ | 2 1 ½ | 2 1 ½ | 2 1 ½ | 2 1 ½ | 2 1 ½ | 2 1 ½ | 2 1 ½ | 2 1 ½ | 2 1 ½ |
| Pflanze | | | | | | | | | | | |
| Gerste | 2 8 9 | 3 4 9 | 2 2 2 | 2 3 7 | 7 9 9 | 6 9 9 | 3 6 9 | 2 3 9 | 2 6 8 | 1 2 3 | 2 4 6 |
| Weizen | 2 8 9 | 6 9 9 | 2 2 3 | 1 2 7 | 9 9 9 | 9 9 9 | 6 9 9 | 2 4 9 | 3 8 9 | 2 3 8 | 3 9 9 |
| Mais | 6 8 9 | 3 7 9 | 2 5 7 | 2 3 4 | 9 9 9 | 9 9 9 | 6 6 9 | 6 8 9 | 5 9 9 | 4 6 9 | 2 4 6 |
| Kulturhirse | 6 9 9 | 9 9 9 | 3 7 8 | 2 3 6 | 9 9 9 | 9 9 9 | 4 7 8 | 5 7 9 | 4 9 9 | 1 4 6 | 1 6 9 |
| Soja | 2 4 7 | 2 3 7 | 3 3 4 | 3 4 6 | 3 5 9 | 7 9 9 | 2 2 5 | 2 5 7 | 1 1 4 | 1 1 5 | 1 3 4 |
| Baumwolle | 1 1 2 | 2 4 7 | 2 2 2 | 1 1 2 | 8 9 9 | 7 8 9 | 6 8 9 | 8 9 9 | 2 7 8 | 2 2 2 | 2 2 6 |
| avena fatua | 1 2 3 | 1 7 9 | 1 1 1 | 1 1 4 | 1 3 9 | 2 8 9 | 2 3 9 | 1 2 4 | 1 3 9 | 1 2 9 | 1 1 8 |
| bromus tectorum | 1 2 3 | 4 5 5 | 2 5 6 | 2 2 2 | 9 9 9 | 9 9 9 | 5 7 9 | 2 3 4 | 1 2 3 | 2 2 9 | 2 4 9 |
| lolium perenne | 2 3 5 | 3 4 9 | 1 2 2 | 1 2 3 | 7 8 9 | 9 9 9 | 5 7 9 | 2 3 6 | 1 3 6 | 2 4 7 | 2 2 4 |
| alopecurus myos. | 1 2 3 | 2 3 4 | 1 1 1 | 1 1 2 | 1 1 6 | 2 2 8 | 2 2 4 | 2 2 4 | 1 2 4 | 1 1 3 | 2 2 4 |
| digiraria sang. | 1 1 2 | 2 2 2 | 1 1 1 | 1 1 2 | 1 2 9 | 2 3 9 | 2 2 3 | 2 4 5 | 1 2 3 | 1 1 6 | 1 2 4 |
| echinochloa crus galli | 1 2 3 | 1 6 7 | 1 1 1 | 1 1 2 | 1 9 9 | 2 7 9 | 2 2 4 | 2 3 6 | 1 2 4 | 1 2 2 | 1 2 4 |
| abatilon | 1 1 1 | 1 1 1 | 1 1 1 | 1 1 1 | 2 2 4 | 1 4 6 | 1 2 2 | 1 1 4 | 1 1 4 | 1 1 1 | 1 2 4 |
| sida spinosa | 1 1 1 | 1 1 1 | 1 3 6 | 1 1 2 | 2 3 3 | 1 4 6 | 1 4 6 | 1 1 6 | 2 2 3 | 1 2 3 | 1 2 3 |
| xanthium | – – – | – – – | – – – | – – – | 2 3 3 | 1 6 9 | 1 1 1 | 1 1 2 | 1 1 1 | 1 1 5 | 1 1 1 |
| amarantus retrofl. | 1 1 1 | 1 1 1 | 1 1 1 | 1 1 1 | 1 2 3 | 1 1 1 | 1 1 1 | 1 1 1 | 1 1 3 | 1 1 1 | 1 1 1 |
| chenopodium album | – – – | – – – | – – – | – – – | 1 1 1 | 1 1 3 | 1 6 7 | 1 1 1 | 2 6 9 | 1 1 1 | 1 1 1 |
| solanum nigrum | 1 1 1 | 1 1 1 | 1 1 1 | 1 1 1 | 1 1 1 | 1 1 1 | 1 1 1 | 1 2 3 | 1 1 1 | 1 1 1 | 1 1 1 |
| ipomoea purpurea | 1 1 3 | 2 3 8 | 1 2 2 | 1 1 3 | 1 3 8 | 2 7 9 | 1 2 3 | 1 2 3 | 1 1 1 | 1 1 2 | 1 1 1 |
| sinapis alba | 1 1 1 | 1 1 3 | 1 1 1 | 1 1 1 | 1 1 6 | 1 1 3 | 1 1 1 | 1 1 2 | 1 1 1 | 1 1 1 | 1 1 1 |
| stellaria media | 1 1 1 | 1 1 1 | 1 1 1 | 1 1 1 | 1 1 2 | 1 2 2 | 1 1 2 | 1 1 1 | 1 1 1 | 1 1 1 | 1 1 2 |
| chrysanthemum leuc. | 1 1 1 | 1 1 1 | 1 1 1 | 1 1 1 | 1 1 1 | 1 7 7 | 1 1 2 | 1 1 1 | 1 1 4 | 1 1 1 | 1 1 1 |
| galium aparine | 1 2 2 | 9 9 9 | 6 7 9 | 1 1 1 | 7 9 9 | 2 6 9 | 1 7 9 | 2 9 9 | 1 1 1 | 1 1 2 | 2 9 9 |
| viola tricolor | – – – | – – – | – – – | – – – | 1 1 9 | 2 3 9 | 4 7 8 | 1 2 6 | 1 1 2 | 1 1 1 | 2 3 6 |
| veronica | – – – | – – – | – – – | – – – | 1 4 8 | 1 1 3 | 1 1 3 | 1 1 9 | 1 1 1 | 1 1 1 | 1 1 1 |
| portulacca | 1 1 1 | 1 1 1 | 1 1 1 | 1 1 1 | – – – | – – – | – – – | – – – | – – – | – – – | – – – |
| kochi scop. | 1 1 1 | 1 1 1 | 1 1 1 | 1 1 1 | – – – | – – – | – – – | – – – | – – – | – – – | – – – |
| sesbania exalt. | 1 1 1 | 1 1 1 | 1 1 1 | 1 1 1 | – – – | – – – | – – – | – – – | – – – | – – – | – – – |

Beispiel 8

Nachweis der Desikkations- und Defoliationswirkung

In einem Gewächshaus wurden Baumwollpflanzen der Sorte Deltapine in Tontöpfen angezogen. Nach abgeschlossener Kapselbildung wurden sie mit wässrigen Zubereitungen der Wirkstoffe Nr. 1 und 2 in einer Aufwandmenge, die 1, 2, 0, 6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter). Die Verbindungen Nr. 1, 2, 7, 104, 108 sowie 110 liessen nach 2 Wochen bloss noch wenige vetrocknete Blätter an den Pflanzen bei Aufwandmengen von 1,2 und 0,6 kg/ha.

Beispiel 9

Nachweis der wuchsregulierenden und ertragssteigernden Wirkung an Sojabohnen

In einer Klimakammer im Gewächshaus werden Sojabohnen der Sorte «Hark» in Kunststoffbehältern in ein Erde-Torf-Sand-Gemisch (6:3:1) ausgesät. Durch optimale Kontrolle der Temperatur, Bewässerung und Beleuchtung sowie durch Düngergabe konnten sich nach ca. 5 Wochen Pflänzchen im 5–6 Trifola-Blattstadium entwikkeln. In diesem Zeitpunkt werden sie mit wässrigen Brühen eines Wirkstoffes in Konzentrationen von 10, 50, 100 und 500 ppm bis zur guten Benetzung (Beginn des Abtropfens) besprüht. Die

Pflanzen werden dann im Gewächshaus weitergezogen. Der Versuch wird 5 Wochen nach der Behandlung ausgewertet.

Gegenüber unbehandelten Kontrollpflanzen zeigten je 10 mit Verbindung No. 6 behandelte Sojapflanzen bei Aufwandmengen von 50 und 100 ppm

- eine Erhöhung des Gewichtes der abgeernteten Schoten um 3 resp. 7%
- eine Verminderung der Wuchshöhe von 9 resp. 14%
- eine durchschnittliche Verlängerung der Seitentriebbildung von 5–11%.

**Patentansprüche**

1. Thiocarbamoylalkoxy-phenyl-harnstoffe der allgemeinen Formel I

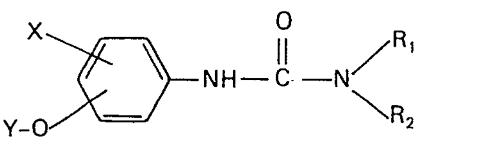

(I)

worin

X Wasserstoff, Halogen, $C_1$–$C_2$-Halogenalkoxy oder $C_1$–$C_2$-Halogenalkyl,

Y eine Thiocarbamoylalkylgruppe der Formel

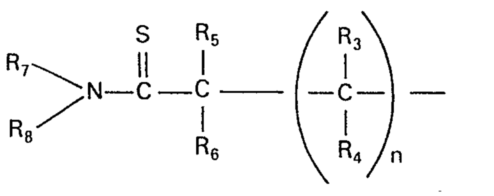

,

n Null, eins oder zwei,

$R_1$ Wasserstoff, $C_1$-$_4$-Alkyl, $C_2$–$C_4$-Alkenyl, $C_2$–$C_4$-Alkinyl oder $C_1$–$C_4$-Alkoxy,

$R_2$ Wasserstoff, $C_1$–$C_4$-Alkyl oder $C_2$–$C_4$-Alkenyl,

$R_3$, $R_4$ und $R_6$ unabhängig voneinander jeweils Wasserstoff oder $C_1$–$C_4$-Alkyl,

$R_5$ Wasserstoff, $C_1$–$C_4$-Alkyl, $C_1$–$C_4$-Alkoxy oder $C_2$–$C_5$-Alkoxy-alkyl,

$R_7$ Wasserstoff, $C_1$–$C_4$-Alkyl, $C_2$–$C_4$-Alkenyl, $C_2$–$C$-$_4$-Alkinyl, $C_1$–$C_4$-Alkoxy oder $C_1$–$C_2$-Alkylphenyl und

$R_8$ Wasserstoff oder $C_1$–$C_4$-Alkyl bedeuten, wobei die Reste $R_1$ und $R_2$ oder $R_7$ und $R_8$ zusammen mit dem sie tragenden Stickstoffatom einen gegebenenfalls durch weitere Heteroatome unterbrochenen 5- bis 6gliedrigen Heterocyclus bilden können.

2. Verbindungen der Formel I, gemäss Anspruch 1, dadurch gekennzeichnet, dass der Substituent Y-O- die meta- oder para-Stellung des Phenylkerns besetzt.

3. Verbindungen der Formel I, gemäss Anspruch 1, dadurch gekennzeichnet, dass die Zahl n Null ist.

4. Thiocarbamoylalkoxy-phenyl-harnstoffe der Formel I, gemäss Anspruch 1, dadurch gekennzeichnet, dass X Wasserstoff oder Halogen bedeutet.

5. Verbindungen der Formel I, gemäss Anspruch 1, dadurch gekennzeichnet, dass der Rest Y-O- die meta-Stellung des Phenylkerns besetzt.

6. Verbindungen der Formel I, gemäss Anspruch 1, dadurch gekennzeichnet, dass der Rest $R_1$ Methyl oder Methoxy und $R_2$ Methyl bedeutet.

7. Thiocarbamoylalkoxy-phenyl-harnstoffe der Formel I, gemäss Anspruch 1, dadurch gekennzeichnet, dass $R_7$ und $R_8$ Wasserstoffatome sind.

8. Thiocarbamoylalkoxy-phenyl-harnstoffe der Formel I, gemäss Anspruch 1, dadurch gekennzeichnet, dass der Substituent Y-O- die meta-Stellung des Phenylkerns besetzt, n Null ist, die Reste X, $R_7$ und $R_8$ Wasserstoff, $R_2$ methyl und $R_1$ Methoxy oder Methyl bedeuten.

9. α-[m-(3-Methoxy-3-methyl-ureido)-phenoxy]-thiopropionsäureamid gemäss Anspruch 1.

10. α-[m-(3,3-Dimethyl-ureido)-phenoxy]-thio-isobuttersäureamid gemäss Anspruch 1.

11. α-[m-(3-Methoxy-3′-methyl-ureido)-phenoxy]-thio-isobuttersäureamid, gemäss Anspruch 1.

12. Verfahren zur Herstellung der Verbindungen der Formel I, dadurch gekennzeichnet, dass man ein Carbonsäurenitril der Formel II

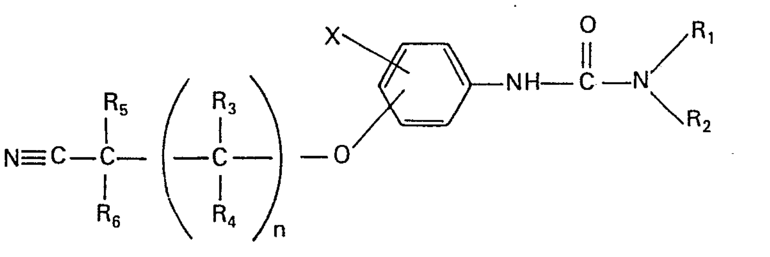

(II)

worin X, n und R1 bis $R_6$ die unter Formel I, Anspruch 1, gegebene Bedeutung haben, gegebenenfalls in Gegenwart eines Katalysators mit Schwefelwasserstoff umsetzt und gegebenenfalls in das substituierte Amid überführt.

13. Ein herbizides Mittel, dadurch gekennzeichnet, dass es neben Träger- und/oder anderen Zuschlagstoffen als Wirkstoff mindestens eine Verbindung der Formel I Anspruch 1 enthält.

14. Die Verwendung der Thiocarbamoylalkoxy-phenyl-harnstoffe gemäss Anspruch 1 oder sie enthaltender Mittel zur Bekämpfung von Unkräutern in Nutzpflanzenkulturen.

15. Die Verwendung der Thiocarbamoylalkoxy-phenyl-harnstoffe gemäss Anspruch 1 zur post-emergenten Bekämpfung von Unkräutern in Soja, Baumwolle, Getreide, Mais, Hirse und Reis.

16. Die Verwendung der Thiocarbamoylalkoxy-phenyl-harnstoffe gemäss Anspruch 1 zur Defoliation und Desikkation von Baumwoll- und Kartoffelkulturen kurz bevor deren Ernte.

17. Die Verwendung der Thiocarbamoylalkoxy-phenyl-harnstoffe gemäss Anspruch 1 zur Hemmung des vegetativen Wachstums von dicotylen Pflanzen.

**Claims**

1. A thiocarbamoylalkoxyphenylurea of the general formula

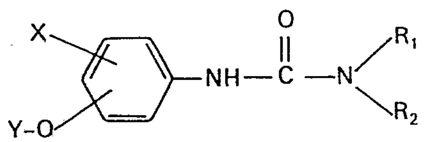

(I)

wherein

X is hydrogen, halogen, $C_1$–$C_2$haloalkoxy or $C_1$–$C_2$haloalkyl,

Y is a thiocarbamoylalkyl group of the formula

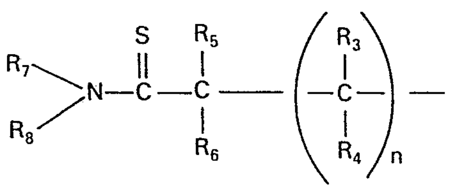

,

n is 0, 1 or 2,

$R_1$ is hydrogen, $C_1$–$C_4$alkyl, $C_2$–$C_4$alkenyl, $C_2$–$C_4$alkynyl or $C_1$–$C_4$alkoxy,

$R_2$ is hydrogen, $C_1$–$C_4$alkyl or $C_2$–$C_4$alkenyl,

$R_3$, $R_4$ and $R_6$, each independently or the other, are hydrogen or $C_1$–$C_4$alkyl,

$R_5$ is hydrogen, $C_1$–$C_4$alkyl, $C_1$–$C_4$alkoxy or $C_2$–$C_5$alkoxyalkyl,

$R_7$ is hydrogen, $C_1$–$C_4$alkyl, $C_2$–$C_4$alkenyl, $C_2$–$C_4$alkynyl, $C_1$–$C_4$-alkoxy or $C_1$–$C_2$alkylphenyl, and

$R_8$ is hydrogen or $C_1$–$C_4$alkyl, whilst $R_1$ and $R_2$ or $R_7$ and $R_8$, together with the nitrogen atom to which they are attached, can form a 5- or 6-membered heterocyclic ring system which can be interrupted by further hetero-atoms.

2. A compound of the formula I according to claim 1, wherein the substituent Y–O– is in the meta- or para-position of the phenyl nucleus.

3. A compound of the formula I according to claim 1, wherein n is 0.

4. A thiocarbamoylalkoxyphenylurea of the formula I according to claim 1, wherein X is hydrogen or halogen.

5. A compound of the formula I according to claim 1, wherein the radical Y–O– is in the meta-position of the phenyl nucleus.

6. A compound of the formula I according to claim 1, wherein $R_1$ is methyl or methoxy and $R_2$ is methyl.

7. A thiocarbamoylalkoxyphenylurea of the formula I according to claim 1, wherein $R_7$ and $R_8$ are hydrogen atoms.

8. A thiocarbamoylalkoxyphenylurea of the formula I according to claim 1, wherein Y–O– is in the meta position of the phenyl nucleus, n is 0, each of X, $R_7$ and $R_8$ is hydrogen, $R_2$ is methyl, and $R_1$ is methoxy or methyl.

9. α-[m-(3-Methoxy-3-methylureido)phenoxy]thiopropionamide according to claim 1.

10. α-[m-(3,3-Dimethylureido)phenoxy]thioisobutyramide according to claim 1.

11. α-[m-(3-Methoxy-3′-methylureido)phenoxy]thioisobutyramide according to claim 1.

12. A process for the production of a compound of the formula I, which process comprises reacting a carboxylic acid nitrile of the formula II

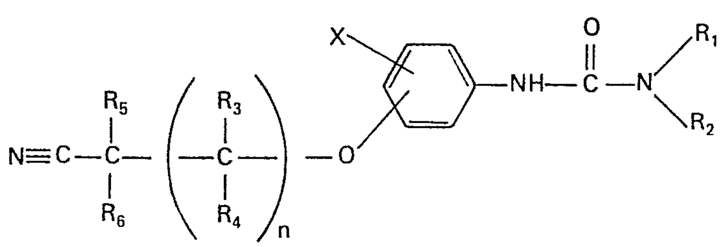

(II)

wherein X, n and $R_1$ to $R_6$ are as defined for formula I in claim 1, optionally in the presence of a catalyst, with hydrogen sulfide, and, if desired, converting the resultant thiocarboxamide into the substituted amide.

13. A herbicidal composition which contains at least one compound of the formula I according to claim 1, together with carriers and/or other adjuvants.

14. A method of controlling weeds in crops of useful plants, which method comprises applying to said crops or to the locus thereof a herbicidally effective amount of a thiocarbamoylalkoxyphenylurea according to claim 1 or of a composition containing such a compound.

15. A method of controlling weeds postemergence in soybeans, cotton, cereals, maize, millet and rice, which method comprises applying thereto a herbicidally effective of a thiocarbamoylalkoxyphenylurea according to claim 1.

16. A method of defoliating and desiccating cotton and potatoes shortly before harvesting, which method comprises applying thereto an effective amount of a thiocarbamoylalkoxyphenylurea according to claim 1.

17. A method of inhibiting the vegetative growth of dicot plants, which method comprises applying thereto an effective amount of a thiocarbamoylalkoxyphenylurea according to claim 1.

**Revendications**

1. Thiocarbamoylalcoxy-phényl-urée de formule générale:

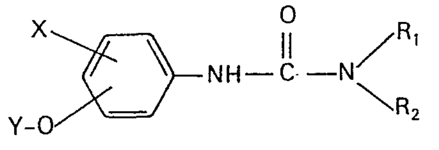

(I)

dans laquelle:
X représente l'hydrogène, un halogène, un groupe halogénoalcoxy en $C_1$–$C_2$ ou halogénoalkyle en $C_1$–$C_2$,
Y représente un groupe thiocarbamoylalkyle de formule:

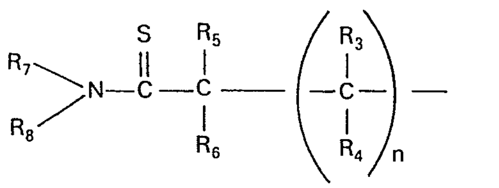

n est égal à 0, 1 ou 2,
$R_1$ représente l'hydrogène, un groupe alkyle en $C_1$–$C_4$, alcényle en $C_2$–$C_4$, alcynyle en $C_2$–$C_4$ ou alcoxy en $C_1$–$C_4$,
$R_2$ représente l'hydrogène, un groupe alkyle en $C_1$–$C_4$ ou alcényle en $C_2$–$C_4$,
$R_3$, $R_4$ et $R_6$ représentent chacun, indépendamment les uns des autres, l'hydrogène ou un groupe alkyle en $C_1$–$C_4$,
$R_5$ représente l'hydrogène, un groupe alkyle en $C_1$–$C_4$, alcoxy en $C_1$–$C_4$ ou alcoxyalkyle en $C_2$–$C_5$,
$R_7$ représente l'hydrogène, un groupe alkyle en $C_1$–$C_4$, alcényle en $C_2$–$C_4$, alcynyle en $C_2$–$C_4$, alkoxy en $C_1$–$C_4$ ou (alkyle en $C_1$–$C_2$)-phényle, et
$R_8$ représente l'hydrogène ou un groupe alkyle en $C_1$–$C_4$,
les restes $R_1$ et $R_2$ ou $R_7$ et $R_8$ pouvant également former avec l'atome d'azote sur lequel ils sont fixés, un hétérocycle à 5–6 chaînons éventuellement interrompu par d'autres hétéroatomes.

2. Composés de formule I selon la revendication 1, caractérisés en ce que le substituant Y-O- occupe la position méta ou para du noyau phényle.

3. Composés de formule I selon la revendication 1, caractérisés en ce que n est égal à 0.

4. Thiocarbamoylalcoxy-phényl-urées de formule I selon la revendication 1, caractérisées en ce que X représente l'hydrogène ou un halogène.

5. Composés de formule I selon la revendication 1, caractérisés en ce que le reste Y-O- occupe la position méta du noyau phényle.

6. Composés de formule I selon la revendication 1, caractérisés en ce que $R_1$, représente un groupe méthyle ou méthoxy et $R_2$ représente un groupe méthyle.

7. Thiocarbamoylalcoxy-phényl-urées de formule I selon la revendication 1, caractérisées en ce que $R_7$ et $R_8$ représentent des atomes d'hydrogène.

8. Thiocarbamoylalcoxy-phényl-urées de formule I selon la revendication 1, caractérisées en ce que le substituant Y-O- occupe la position méta du noyau phényle, n est égal à 0, les symboles X, $R_7$ et $R_8$ représentent l'hydrogène, $R_2$ le groupe méthyle et $R_1$ un groupe méthoxy ou méthyle.

9. L'alpha-[m-(3-méthoxy-3-méthyl-uréido)-phénoxy]-thiopropionamide selon la revendication 1.

10. L'alpha-[m-(3,3-diméthyl-uréido)-phénoxy]-thioisobutyramide selon la revendication 1.

11. L'alpha-[m-(3-méthoxy-3'-méthyl-uréido)-phénoxy]-thioisobutyramide selon la revendication 1.

12. Procédé de préparation des composés de formule I, caractérisé en ce que l'on fait réagir un nitrile d'acide carboxylique de formule II:

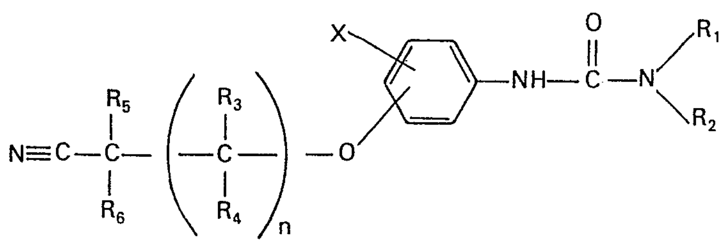

(II)

dans laquelle X, n et $R_1$ à $R_6$ ont les significations indiquées en référence à la formule I, revendication 1, éventuellement en présence d'un catalyseur, avec le sulfure d'hydrogène, et le cas échéant on convertit en l'amide substitué.

13. Produit herbicide caractérisé en ce qu'il contient en tant que substance active, avec des véhicules et/ou d'autres additifs, au moins un composé de formule I selon la revendication 1.

14. Utilisation des thiocarbamoylalcoxy-phénylurées selon la revendication 1 ou de produits en contenant dans la lutte contre les mauvaises herbes dans des cultures de végétaux utiles.

15. Utilisation des thiocarbamoylalcoxy-phénylurées selon la revendication 1 dans la lutte en post-levée contre les mauvaises herbes dans les cultures de soja, de coton, de céréales, de maïs, de millet et de riz.

16. Utilisation des thiocarbamoylalcoxy-phényl-urées selon la revendication 1, pour la défoliation et la dessiccation des cultures de coton et de pommes de terre peu avant leur récolte.

17. Utilisation des thiocarbamoylalcoxy-phényl-urées selon la revendication 1, pour l'inhibition de la croissance végétative des végétaux dicotylédones.